# EUROPÄISCHE PATENTSCHRIFT

(11) **EP 3 161 477 B1**
(45) Veröffentlichungstag und Bekanntmachung des Hinweises auf die Patenterteilung: **03.01.2018**
(21) Anmeldenummer: 16705015.2
(22) Anmeldetag: 26.01.2016
(51) Int. Cl.: G01N 33/36, G01D 11/24

(54) **MODULARES GARNPRÜFGERÄT**
MODULAR YARN TESTER
APPAREIL MODULAIRE DE CONTRÔLE DE FIL

(30) Priorität: 20.03.2015 CH 4072015
(43) Veröffentlichungstag der Anmeldung: 03.05.2017
(73) Patentinhaber: Uster Technologies AG, 8610 Uster (CH)
(72) Erfinder: DE VRIES, Loris, 8625 Gossau (CH); FENNER, Jürg, 8600 Dübendorf (CH)
(74) Vertreter: Pliska, Pavel
(86) Internationale Anmeldenummer: PCT/CH2016/000017
(87) Internationale Veröffentlichungsnummer: WO 2016/149839

(56) Entgegenhaltungen:
- DE-A1-102009 057 129
- GB-A- 2 192 722
- US-A- 4 845 983

## Beschreibung

### FACHGEBIET

Die vorliegende Erfindung liegt auf dem Gebiet der textilen Qualitätsprüfung und bezieht sich auf ein modulares Prüfgerät zur Prüfung von länglichem textilem Prüfgut wie Garn, Vorgarn oder Faserband, gemäss dem Oberbegriff des ersten Patentanspruchs. Das erfindungsgemässe Prüfgerät ist als eigenständige, nicht in einen Produktionsprozess eingebundene Vorrichtung konzipiert und somit im Off-line-Betrieb, bspw. im Textillabor, einsetzbar.

### STAND DER TECHNIK

Prüfgeräte dieser Art werden dort eingesetzt, wo die Qualität von Garnen oder anderen in Faden- oder Bandform vorliegenden textilen Materialien geprüft werden muss. Es geht darum, einerseits eine gleich bleibende Qualität der herzustellenden textilen Produkte zu erreichen, andererseits aber auch darum, den textilen Produktionsprozess anhand von Stichproben off-line zu überwachen.

Geprüft und protokolliert werden mit Prüfgeräten dieser Art in der Regel eine ganze Reihe unterschiedlicher Parameter des zu prüfenden textilen Prüfgutes. Die Anzahl der zu prüfenden Parameter und damit natürlich auch die Komplexität des dazu notwendigen Prüfgerätes hängen von verschiedenen Faktoren ab, so beispielsweise davon, ob Stapel-oder Filamentgarne geprüft werden müssen, aber auch davon, welche Qualitätsstandards für das Textilprodukt vorgegeben sind. Zu den interessierenden Parametern gehört heute in der Regel zumindest die Massenungleichmässigkeit, die mit einem kapazitiven Sensor gemessen wird. Oftmals und gerade bei Naturgarnen kommen aber auch noch optische Sensoren beispielsweise zur Messung von Haarigkeit, Haarigkeitslängen, Fremdstoffe etc. zum Einsatz. Verschiedene weitere interessierende Parameter können dazu treten.

Die bekannten Prüfgeräte dieser Art arbeiten nach dem Durchlaufprinzip, d. h. das zu prüfende Garn wird in einem einzigen Prüfdurchlauf von einer Garnspule abgezogen und durchläuft in serieller Weise im Prüfgerät eine Anordnung von Sensoren, um die zu bestimmenden Parameter zu messen. Dies ist grundsätzlich ein kontinuierlicher Prozess. Je nach Automatisierungsgrad ist auch die mechanische Erfassung und Einführung des Prüfgutes in das Prüfgerät in demselben realisiert. Die Auswertung und statistische Analyse der Sensorsignale erfolgen in der Regel in einer mit dem Prüfgerät verbundenen Steuer- und Auswerteeinheit. Diese interessiert im Kontext der vorliegenden Erfindung jedoch nicht.

Zur Vermeidung messverfälschender Ablagerungen von Fasern, Staub und anderen Verunreinigungen an den Sensoren wurden bereits unterschiedliche Massnahmen vorgeschlagen. Diese nutzen prinzipiell eine künstlich erzeugte Luftströmung.

Die GB-2'192'722 A offenbart ein Garnprüfgerät mit mehreren Messmodulen (5, 8) in einem turmartigen Aufbau, sodass sie entlang der Front des Prüfgerätes einen Prüfgutpfad definieren.

Die CH-563 021 A5 beschreibt ein Verfahren zur Erzielung eines stabilen elektrischen Verhaltens des Messorgans von Textilprüfgeräten während des Betriebs sowie eine Vorrichtung zur Durchführung des Verfahrens. Bevorzugt wird dabei eine Luftströmung über die Oberseite des Messorgans geführt, und zwar in einer Weise, dass das Messorgan von der Umgebungsluft infolge Luftkonvektion vermehrt umströmt wird. Ablagerungen an den Messorganen werden so verhindert, die Staub- und Faserteile werden jedoch in die Umgebungsluft ausgeblasen. Es handelt sich hier um ein einfaches, nicht erweiterbares Prüfgerät mit Luftansaugung über ein Luftfilter in einer seitlichen Wand.

Die US-D412290S wie auch die US-D412291S zeigen jeweils ein Garnprüfgerät mit einer automatisierten Einführungsvorrichtung und einem halbautomatischer Garnwechsler. Aus den Rückseitenansichten der beiden Designschriften ist klar ersichtlich, dass jedes Funktionsmodul einen eigenen eingebauten Ventilator hat.

Die US-4,845,983 A zeigt ein Modul eines Gleichmässigkeitsprüfers zur Bestimmung der Masseschwankungen von textilem Prüfgut. An der Rückwand des Moduls ist ein Ventilator zur Kühlung und Belüftung des Innenteils des Moduls montiert.

Die WO-2007/014475 A1 offenbart ein Garnprüfgerät, bei dem ebenfalls eine automatisierte Einführungsvorrichtung und ein halbautomatischer Garnwechsler vorhanden sind. Um Ablagerungen von losen Fasern, Staub- oder Schmutzteilchen zu verringern, sind mehrere entlang des Messpfades angeordnete und voneinander beabstandete Absaugorgane vorgesehen, die einen Unterdruck erzeugen. Im Unterschied zur vorher genannten CH-563 021 A5 werden die unerwünschten Partikel also mittels des Unterdrucks abgesaugt und gelangen nicht in die Umgebungsluft. Die Anordnung und die Mittel zur Erzeugung des Unterdrucks sind allerdings nicht offenbart.
Die im Stand der Technik beschriebenen Lösungen haben also, sofern es sich um Lösungen mit Staubausblasung handelt, pro Funktionsmodul (Basis- oder Optionsmodul) jeweils einen separaten Ventilator. Damit wird zwar pro Funktionsmodul stets eine adäquate Ausblaskapazität zur Verfügung gestellt, die Lösung erfordert aber im Falle einer grösseren Anzahl von optionalen Funktionsmodulen auch eine grössere Anzahl von verbauten Ventilatoren. Das ist teuer und erzeugt laute Ventilatorgeräusche. Zudem sind die einzelnen Ventilatoren wegen der erforderlichen kompakten Bauweise in der Regel nicht besonders langlebig.

Die DE-33'16'978 A1 bezieht sich auf ein Aufbausystem zur Abführung der Verlustwärme von in Behältern oder Schränken angeordneten elektronischen Baugruppen. Für das Aufbausystem werden stapelbare, kastenförmige Baugruppenträger verwendet, die an ihren Rückseiten mit zwei Einlassöffnungen und an ihren Frontseiten mit Auslassschlitzen für den Kühlluftstrom versehen sind. Die Lüfteraggregate bestehen aus rohrartigen Luftkanälen mit in Fortsätzen untergebrachten Radialgebläsen. Die Luftkanäle sind mit symmetrisch zu einer Mantellinie angebrachten Öffnungen, die hinsichtlich Abstand und Abmessungen zu den Einlassöffnungen in den gestapelten Baugruppenträgern passend mit diesen gasleitend verbunden sind.

### DARSTELLUNG DER ERFINDUNG

Der Markt fordert Garnprüfgeräte, die den unterschiedlichsten Anforderungen in Bezug auf die Menge messbarer Parameter gerecht werden und die auch bei einer Erweiterung der Menge messbarer Parameter keinen unverhältnismässig grossen Anpassungsaufwand erfordern. Da besonders bei der Prüfung von Stapelgarnen eine starke Anfälligkeit für Staub- und Faseransammlungen besteht, welche die Genauigkeit und die Zuverlässigkeit der Sensoren beeinflussen, müssen Mittel vorgesehen sein, um messverfälschende Ablagerungen an den Sensoren möglichst zu verhindern. Die Wirksamkeit der getroffenen Massnahmen muss auch bei einer Vielzahl verschiedener Sensoren gewährleistet sein.

Es ist eine Aufgabe der vorliegenden Erfindung, ein modulares Prüfgerät zur Prüfung von länglichem textilem Prüfgut wie Garn, Vorgarn oder Faserband mit Mitteln zur Vermeidung von Ablagerungen zur Verfügung zu stellen, das leichter an geänderte Umstände anpassbar, kostengünstiger, leiser, zuverlässiger und langlebiger ist als die aus dem Stand der Technik bekannten Prüfgeräte.

Diese und andere Aufgaben werden durch das erfindungsgemässe modulare Prüfgerät, wie es im ersten Patentanspruch definiert ist, gelöst. Vorteilhafte Ausführungsformen sind in den abhängigen Patentansprüchen angegeben.

Der Erfindung liegt die Idee zugrunde, mehrere Funktionsmodule des modularen Prüfgerätes zu einer Belüftungsgruppe zusammenzufassen und für die Belüftungsgruppe eine zentrale Belüftungseinheit mit dem Funktionsprinzip der Staubausblasung vorzusehen. Ansonsten wird der an sich bekannte turmartige Aufbau des modularen Prüfgerätes mit einer Mehrzahl von Funktionsmodulen zur Messung verschiedener Parameter des Prüfgutes beibehalten. Somit wird ein gemeinsames Innenvolumen der Belüftungsgruppe Aufbaus inklusive der zusammengefassten Funktionsmodule von der zentralen Belüftungseinheit mit Ausblasluft versorgt.

Wenn alle Funktionsmodule des turmartigen Aufbaus eine einzige Belüftungsgruppe bilden, ist die zentrale Belüftungseinheit ein Teil einer den Funktionsmodulen gemeinsamen Infrastruktur des Prüfgerätes. Weitere Teile der gemeinsamen Infrastruktur können z. B. eine Fördereinrichtung für das textile Prüfgut, eine Absaugöffnung für das textile Prüfgut, eine Spannungsversorgung, eine Garnwechselvorrichtung und/oder eine Garneinführungsvorrichtung sein.

Das modulare Prüfgerät dient zur Prüfung von länglichem textilem Prüfgut wie Garn, Vorgarn oder Faserband. Das Prüfgerät weist einen Prüfgutpfad und einen turmartigen Aufbau mit einer Mehrzahl entlang des Prüfgutpfades nacheinander angeordneter Funktionsmodule zur Messung verschiedener Parameter des Prüfgutes auf. Mehrere Funktionsmodule innerhalb des turmartigen Aufbaus sind gasleitend direkt miteinander verbunden, so dass sie zusammen eine Belüftungsgruppe bilden, in deren Inneren ein gemeinsames Innenvolumen vorhanden ist. Die Belüftungsgruppe weist eine zentrale Belüftungseinheit auf, die für die Belüftung aller Funktionsmodule der Belüftungsgruppe ausgebildet ist und die mindestens einen Zentralventilator zum Aufbau eines Luftüberdrucks in dem gemeinsamen Innenvolumen beinhaltet.

In einer Ausführungsform bilden alle Funktionsmodule des turmartigen Aufbaus eine einzige Belüftungsgruppe.

In einer Ausführungsform befindet sich die zentrale Belüftungseinheit im gemeinsamen Innenvolumen.

In einer Ausführungsform verläuft der Prüfgutpfad entlang einer Front des Prüfgerätes und die zentrale Belüftungseinheit ist in einer Wand des turmartigen Aufbaus, die von der Front verschieden ist. eingebaut. Die Wand, in welcher die zentrale Belüftungseinheit eingebaut ist, ist vorzugsweise eine der Front gegenüberliegende Rückwand des turmartigen Aufbaus.

Ein Lufteinlass des mindestens einen Zentralventilators kann mit einem Gitter, Sieb und/oder Luftfilter zur Verhinderung eines Eindringens von Verunreinigungen in das gemeinsame Innenvolumen versehen sein.

In einer Ausführungsform weist die zentrale Belüftungseinheit mehrere Zentralventilatoren auf. Mehrere Zentralventilatoren werden vorteilhafterweise dann eingesetzt, wenn ein einziger Zentralventilator zu schwach wäre, um die ablagerungsverhindernde Wirkung wirksam zu erzielen. Mehrere Zentralventilatoren in der zentralen Belüftungseinheit eröffnen die Möglichkeit des Zu- und Abschaltens einzelner der Zentralventilatoren, je nach Bedarf. Auf diese Weise lässt sich eine gemeinsame Infrastruktur bereitstellen, die prinzipiell nicht erweiterungsbedürftig ist.

In einer Ausführungsform weist das Prüfgerät eine Grundinfrastruktur zur Bereitstellung einer Grundfunktionalität auf. Der turmartige Aufbau umfasst dann vorzugsweise ein Basisgehäuse für die Grundinfrastruktur und ein auf das Basisgehäuse aufgesetztes Optionengehäuse für optionale Funktionsmodule

Unter dem Begriff "Grundfunktionalität" versteht man dabei solche Funktionen, die selbst einfachste Prüfgeräte zur Prüfung von länglichem textilem Prüfgut sinnvollerweise mindestens aufweisen sollten, um nutzbringend eingesetzt werden zu können. Die Grundinfrastruktur zur Bereitstellung der Grundfunktionalität kann bspw. mindestens eine Fördereinrichtung für das textile Prüfgut, eine Absaugöffnung für das textile Prüfgut und einen Sensor zur Gleichmässigkeitsprüfung des textilen Prüfgutes umfassen.

In einer Ausführungsform bilden alle Funktionsmodule des turmartigen Aufbaus eine einzige Belüftungsgruppe, und die zentrale Belüftungseinheit weist zwei in einem Bereich des Basisgehäuses angebrachte Zentralventilatoren und einen dritten, in einem Bereich des Optionengehäuses angebrachten Zentralventilator auf.

Der turmartige Aufbau kann vom gemeinsamen Innenvolumen nach aussen führende Luftaustrittsöffnungen aufweisen, durch welche der von dem mindestens einen Zentralventilator im gemeinsamen Innenvolumen aufbaubare Luftüberdruck abbaubar ist, so dass an den Luftaustrittsöffnungen nach aussen gerichtete Teilluftströme erzeugbar sind. Zumindest ein Teil der Luftaustrittsöffnungen liegt vorzugsweise im Prüfgutpfad, um mit den nach aussen gerichteten Teilluftströmen Staubablagerungen im Garnpfad und an den Funktionsmodulen zu verhindern.

Die zentrale Belüftungseinheit mit dem mindestens einen darin eingebauten Zentralventilator hat gegenüber den pro Funktionsmodul verbauten Einzelventilatoren den Vorteil, dass einzelne Funktionsmodule leichter ausgetauscht werden können, ohne auf die Belüftungsmittel Rücksicht nehmen zu müssen. Dadurch ist das erfindungsgemässe Prüfgerät leichter an geänderte Umstände anpassbar, einfacher zu warten und zu reparieren. Dank der Möglichkeit des Einsatzes grösserer Zentralventilatoren resultiert eine wesentlich längere Lebensdauer und zugleich auch eine Geräuschreduktion. Mit der so realisierten Staubausblasung kann das Prüfgut auch bei hohen Durchlaufgeschwindigkeiten des Prüfgutes (z. B. bis 800 m/s) sauber und störungsfrei geprüft werden.

### AUFZÄHLUNG DER ZEICHNUNGEN

Nachfolgend wird eine Ausführungsform der Erfindung anhand der Zeichnungen detailliert erläutert.
- Figur 1: zeigt ein erfindungsgemässes modulares Prüfgerät von links vom.
- Figur 2: zeigt das modulare Prüfgerät von Fig. 1 von rechts hinten.
- Figur 3: zeigt das modulare Prüfgerät der Figuren 1 und 2 in derselben Ansicht wie Fig. 2, aber mit entfernter Rückseitenabdeckung.

### AUSFÜHRUNG DER ERFINDUNG

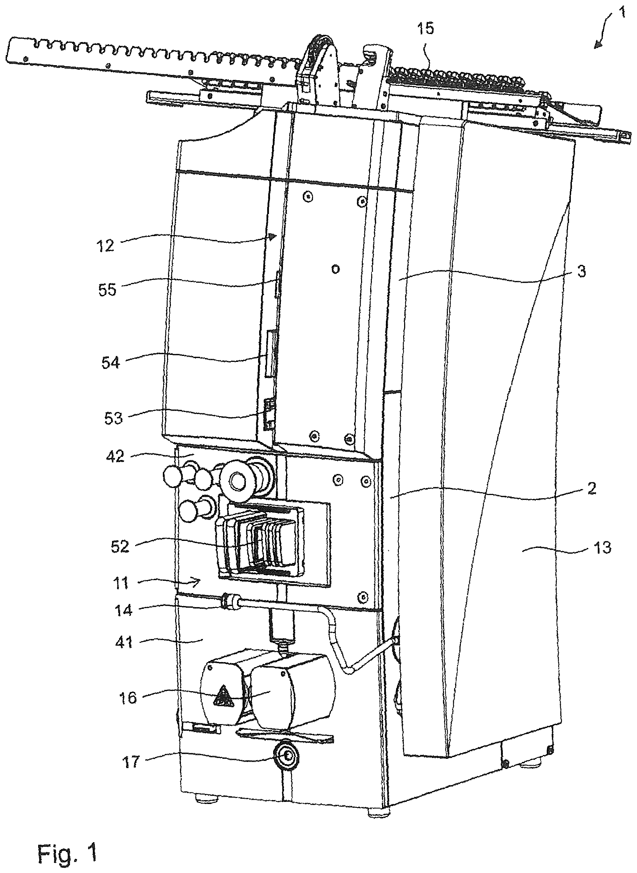

**Figur 1** zeigt beispielhaft eine Ausführungsform des erfindungsgemässen modularen Prüfgerätes 1 in einer Ansicht von links vorn. Das Prüfgerät 1 weist eine Front 11 auf, entlang welcher ein Prüfgutpfad 12 für ein zu prüfendes (nicht eingezeichnetes) textiles Prüfgut verläuft. Auf einer Seite des Prüfgerätes 1, welche von der Front 11 verschieden ist, ist eine automatische Einführungsvorrichtung 13 zum Einführen des Prüfgutes in den Prüfgutpfad 12 angebracht. Zum Einbringen in den Prüfgutpfad 12 wird das Prüfgut von einem verschiebbaren und drehbaren Greifer 14 der Einführungsvorrichtung 13 ergriffen und durch eine entsprechende Bewegung des Greifers 14 eingebracht.

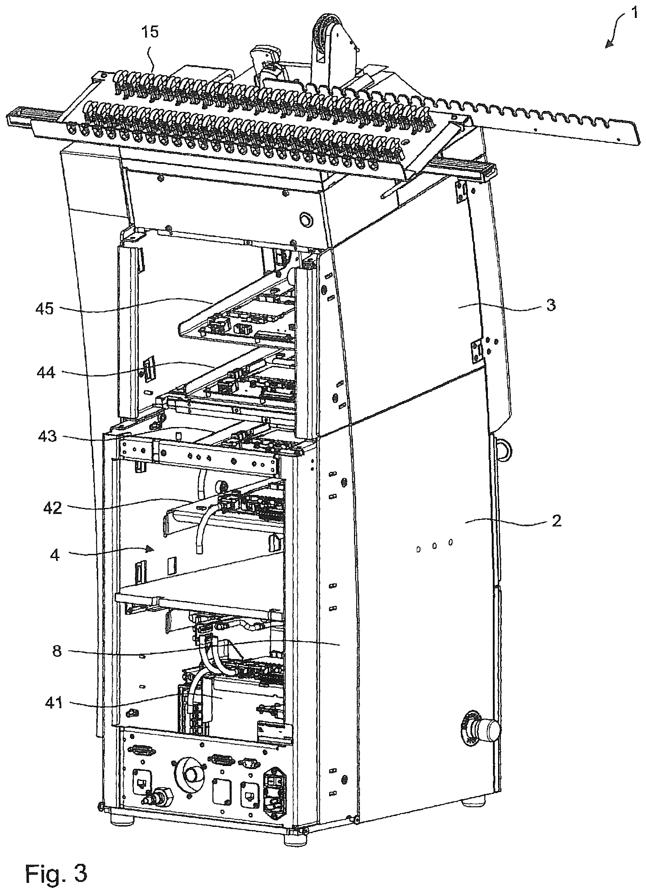

Während des Prüfvorgangs tritt das Prüfgut durch eine automatische Garnwechselvorrichtung 15 in den Prüfgutpfad 12 ein. Im Prüfgutpfad 12 durchläuft das Prüfgut verschiedene Sensoren 52-55, die in verschiedenen Funktionsmodulen 42-45 (siehe Figur 3) eingebaut sind. Das Prüfgut wird von einer Fördereinrichtung 16 entlang seiner Längsrichtung durch den Prüfgutpfad 12 gefördert. Die Fördereinrichtung 16 kann z. B. als Rollenlieferwerk mit zwei zusammenwirkenden Förderrollen von denen mindestens eine zur Rotation angetrieben ist, ausgebildet sein. Schliesslich verlässt das Prüfgut den Prüfgutpfad 12 durch eine Absaugöffnung 17.

Das Prüfgerät 1 hat einen turmartigen Aufbau mit einem Basisgehäuse 2 und einem darauf aufgesetzten Optionengehäuse 3. Das Basisgehäuse 2 und das Optionengehäuse 3 umfassen Funktionsmodule 41-45 (siehe Figur 3) zur Prüfung des länglichen Prüfgutes. Ein im Basisgehäuse 2 eingebautes erstes Funktionsmodul 41 beinhaltet im vorliegenden Fall die Fördereinrichtung 16 und die Absaugöffnung 17. Ein zweites Funktionsmodul 42 beinhaltet mindestens einen kapazitiven Sensor 52 zur Gleichmässigkeitsprüfung des Prüfgutes. Weitere, oberhalb des zweiten Funktionsmoduls 42 befindliche Funktionsmodule 43-45, welche weitere Sensoren 53-55 beinhalten, sind wegen einer Frontabdeckung in Figur 1 nicht sichtbar.

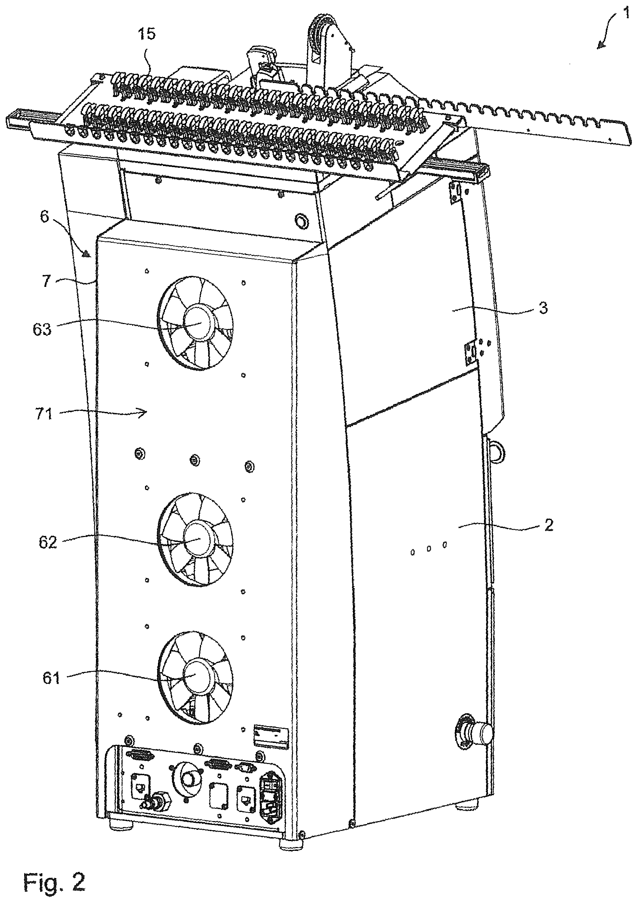

In den **Figuren 2** **und** **3** ist das modulare Prüfgerät 1 von Figur 1 in einer Ansicht von rechts hinten dargestellt, und zwar mit einer Rückseitenabdeckung 7 bzw. ohne Rückseitenabdeckung. In der offengelegten Ansicht von Figur 3 sind insbesondere sowohl im Basisgehäuse 2 als auch im Optionengehäuse 3 eingefügte Funktionsmodule 41-45 sichtbar.

Alle Funktionsmodule 41-45 innerhalb des turmartigen Aufbaus sind gasleitend direkt miteinander verbunden. Die gasleitende direkte Verbindung kommt hauptsächlich in dem durch die Rückseitenabdeckung 7 von drei Seiten umschlossenen Volumen zustande. Weitere gasleitende direkte Verbindungen innerhalb des turmartigen Aufbaus können z. B. dadurch entstehen, dass einzelne Funktionsmodule 41-45 nicht die ganze Querschnittsfläche des turmartigen Aufbaus abdecken, oder dass sie gasleitende Öffnungen aufweisen. Die Funktionsmodule 41-45 bilden somit zusammen eine Belüftungsgruppe, in deren Inneren ein gemeinsames Innenvolumen 4 vorhanden ist.
Eine einstückige Rückwand 71 erstreckt sich im Wesentlichen über eine gesamte der Front 11 gegenüberliegende Rückseite des turmartigen Aufbaus. Die Rückwand 71 ist Teil der einstückigen Rückseitenabdeckung 7. Die Rückseitenabdeckung 7 ist im vorliegenden Ausführungsbeispiel mit Befestigungsmitteln wie Schrauben an einem Rahmen 8 des turmartigen Aufbaus befestigbar.

In die Rückwand 71 ist eine zentrale Belüftungseinheit 6 des Prüfgerätes 1 eingebaut. Im vorliegenden Ausführungsbeispiel beinhaltet die zentrale Belüftungseinheit 6 drei Zentralventilatoren 61-63; Ausführungsformen mit einer grösseren oder kleineren Anzahl Zentralventilatoren gehören ebenfalls zur vorliegenden Erfindung. Ein erster Zentralventilator 61 und ein zweiter Zentralventilator 62 sind im Bereich des Basisgehäuses 2 angebracht, ein dritter Zentralventilator 63 im Bereich des Optionengehäuses 3. Trotz dieser lokalen Anordnung dient jeder der Zentralventilatoren 61-63 der Belüftung aller Funktionsmodule 41-45 der Belüftungsgruppe. Die Zentralventilatoren 61-63 bauen einen Luftüberdruck im gemeinsamen Innenvolumen 4 auf. Im hier diskutierten Ausführungsbeispiel befinden sich die Zentralventilatoren 61-63 und somit die zentrale Belüftungseinheit 6 im gemeinsamen Innenvolumen 4.

Lufteinlässe der Zentralventilatoren 61-63, d. h. die Öffnungen in der Rückwand 71, durch die in Figur 2 die Zentralventilatoren 61-63 sichtbar sind, können mit (nicht eingezeichneten) Luftfiltern versehen sein. Solche Luftfilter verhindern ein Eindringen von Verunreinigungen wie Staub oder textile Fasern in das gemeinsame Innenvolumen 4 verhindern. Alternativ oder zusätzlich zu Luftfiltern können zum gleichen Zweck Gitter und/oder Siebe an den Lufteinlässen angebracht sein.

Der von den Zentralventilatoren 6I-63 im gemeinsamen Innenvolumen 4 aufbaubare Luftüberdruck ist durch nach aussen gerichtete (in den Zeichnungen nicht sichtbare) Luftaustrittsöffnungen abbaubar, die im turmartigen Aufbau vorhanden sind. An den Luftaustrittsöffnungen sind somit nach aussen gerichtete Teilluftströme erzeugbar. Es ist vorteilhaft, Luftaustrittsöffnungen im Prüfgutpfad 12 (siehe Figur 1) und insbesondere im Bereich der Sensoren 52-55 vorzusehen. Die Luftaustrittsöffnungen sind für diesen Zweck speziell geschaffen und entsprechend zweckoptimiert. Ihre Lage, Form, Grösse und Anzahl sind an die jeweilige Stelle, z. B. an den Sensor 52-55, bei dem sie sich befinden, individuell angepasst. Durch die an diesen Stellen austretenden Teilluftströme werden messverfälschende Faser- und Staubablagerungen an den Sensoren 52-55 und im Garnpfad 12 verhindert.

Als Zentralventilatoren 61-63 können bspw. handelsübliche Ventilatoren mit jeweils einem Durchmesser von 120 mm eingesetzt werden. Dieser relativ grosse Durchmesser erlaubt eine robuste Bauweise, Geräuscharmut und Langlebigkeit der Zentralventilatoren 61-63. In der hier diskutierten Ausführungsform bilden alle Funktionsmodule 41-45 des Prüfgerätes 1 eine einzige Belüftungsgruppe. Alternativ kann nur eine Teilmenge aller Funktionsmodule 41-45 eine Belüftungsgruppe bilden, z. B. nur die im Basisgehäuse 2 untergebrachten Funktionsmodule 41, 42 und/oder nur die im Optionengehäuse 3 untergebrachten Funktionsmodule 43-45. Somit kann das Prüfgerät 1 nur eine einzige Belüftungsgruppe oder mehrere Belüftungsgruppen aufweisen.
Selbstverständlich ist die vorliegende Erfindung nicht auf die oben diskutierten Ausführungsformen beschränkt. Bei Kenntnis der Erfindung wird der Fachmann weitere Varianten innerhalb des Schutzumfangs der Ansprüche herleiten können, die auch zum Gegenstand der vorliegenden Erfindung gehören.

### BEZUGSZEICHENLISTE

- 1: Prüfgerät
- 11: Front
- 12: Prüfgutpfad
- 13: Einführungsvorrichtung
- 14: Greifer
- 15: Garnwechselvorrichtung
- 16: Fördereinrichtung
- 17: Absaugöffnung

- 2: Basisgehäuse

- 3: Optionengehäuse

- 4: gemeinsames Innenvolumen
- 41-46: Funktionsmodule

- 52: kapazitive Massesensoren
- 53: optischer Mehrzwecksensor
- 54: optischer Sensor zur Detektion von Verunreinigungen
- 55: optischer Sensor zur Prüfung der Haarigkeit

- 6: zentrale Belüftungseinheit
- 61-63: Zentralventilatoren

- 7: Rückseitenabdeckung
- 71: Rückwand

- 8: Rahmen des turmartigen Aufbaus

## Patentansprüche

1. Modulares Prüfgerät (1) zur Prüfung von länglichem textilem Prüfgut wie Garn, Vorgarn oder Faserband, wobei das Prüfgerät (1)
einen Prüfgutpfad (12) und
einen turmartigen Aufbau mit einer Mehrzahl entlang des Prüfgutpfades (12) nacheinander angeordneter Funktionsmodule (41-45) zur Messung verschiedener Parameter des Prüfgutes
aufweist,
**dadurch gekennzeichnet, dass**
mehrere Funktionsmodule (41-45) innerhalb des turmartigen Aufbaus gasleitend direkt miteinander verbunden sind, so dass sie zusammen eine Belüftungsgruppe bilden, in deren Inneren ein gemeinsames Innenvolumen (4) vorhanden ist, und die Belüftungsgruppe eine zentrale Belüftungseinheit (6) aufweist, die für die Belüftung aller Funktionsmodule (41-45) der Belüftungsgruppe ausgebildet ist und die mindestens einen Zentralventilator (61-63) zum Aufbau eines Luftüberdrucks in dem gemeinsamen Innenvolumen (4) beinhaltet.

2. Modulares Prüfgerät (1) nach Anspruch 1, wobei alle Funktionsmodule (41-45) des turmartigen Aufbaus eine einzige Belüftungsgruppe bilden.

3. Modulares Prüfgerät (1) nach einem der vorangehenden Ansprüche, wobei sich die zentrale Belüftungseinheit (6) im gemeinsamen Innenvolumen (4) befindet.

4. Modulares Prüfgerät (1) nach einem der vorangehenden Ansprüche, wobei der Prüfgutpfad (12) entlang einer Front (11) des Prüfgerätes (1) verläuft und die zentrale Belüftungseinheit (6) in einer Wand (71) des turmartigen Aufbaus, die von der Front (11) verschieden ist, eingebaut ist.

5. Modulares Prüfgerät (1) nach Anspruch 4, wobei die Wand (71), in welcher die zentrale Belüftungseinheit (6) eingebaut ist, eine der Front (11) gegenüberliegende Rückwand des turmartigen Aufbaus ist.

6. Modulares Prüfgerät (1) nach einem der vorangehenden Ansprüche, wobei ein Lufteinlass des mindestens einen Zentralventilators (61-63) mit einem Gitter, Sieb und/oder Luftfilter zur Verhinderung eines Eindringens von Verunreinigungen in das gemeinsame Innenvolumen (4) versehen ist.

7. Modulares Prüfgerät (1) nach einem der vorangehenden Ansprüche, wobei die zentrale Belüftungseinheit (6) mehrere Zentralventilatoren (61-63) aufweist.

8. Modulares Prüfgerät (1) nach einem der vorangehenden Ansprüche, wobei das Prüfgerät (1) eine Grundinfrastruktur zur Bereitstellung einer Grundfunktionalität aufweist und
der turmartige Aufbau ein Basisgehäuse (2) für die Grundinfrastruktur und ein auf das Basisgehäuse (2) aufgesetztes Optionengehäuse (3) für optionale Funktionsmodule (43-45) umfasst.

9. Modulares Prüfgerät (1) nach Anspruch 8, wobei die Grundinfrastruktur zur Bereitstellung der Grundfunktionalität mindestens eine Fördereinrichtung (16) für das textile Prüfgut, eine Absaugöffnung (17) für das textile Prüfgut und einen Sensor (52) zur Gleichmässigkeitsprüfung des textilen Prüfgutes umfasst.

10. Modulares Prüfgerät (1) nach Anspruch 2 und Anspruch 8 oder 9, wobei alle Funktionsmodule (41-45) des turmartigen Aufbaus eine einzige Belüftungsgruppe bilden und die zentrale Belüftungseinheit (6) zwei in einem Bereich des Basisgehäuses (2) angebrachte Zentralventilatoren (61, 62) und einen dritten, in einem Bereich des Optionengehäuses (3) angebrachten Zentralventilator (63) aufweist.

11. Modulares Prüfgerät (1) einem der vorangehenden Ansprüche, wobei der turmartige Aufbau vom gemeinsamen Innenvolumen (4) nach aussen führende Luftaustrittsöffnungen aufweist, durch weIche der von dem mindestens einen Zentralventilator (61-63) im gemeinsamen Innenvolumen (4) aufbaubare Luftüberdruck abbaubar ist, so dass an den Luftaustrittsöffnungen nach aussen gerichtete Teilluftströme erzeugbar sind.

12. Modulares Prüfgerät (1) nach Anspruch 11, wobei zumindest ein Teil der Luftaustrittsöffnungen im Prüfgutpfad (12) liegt, um mit den nach aussen gerichteten Teilluftströmen Staubablagerungen im Prüfgutpfad (12) und an den Funktionsmodulen (41-45) zu verhindern.

## Claims

1. A modular tester (1) for testing elongated textile test material such as yarn, roving or sliver, wherein the tester (1) comprises
a test material path (12) and
a turret-like setup with a plurality of functional modules (41 to 45) which are arranged in succession along the test material path (12) for measuring various parameters of the textile test material,
**characterized in that**
several functional modules (41 to 45) are directly connected to each other in a gas-conducting manner within the turret-like setup, so that they jointly form a ventilation group, in the interior of which a common interior volume (4) is present, and
the ventilation group comprises a central ventilation unit (6) which is formed for the ventilation of all functional modules (41 to 45) of the ventilation group and which contains at least one central fan (61 to 63) for building up an excess air pressure in the common interior volume (4).

2. A modular tester (1) according to claim 1, wherein all functional modules (41 to 45) of the turret-like setup form a single ventilation group.

3. A modular tester (1) according to one of the preceding claims, wherein the central ventilation unit (6) is disposed in the common interior volume (4).

4. A modular tester (1) according to one of the preceding claims, wherein the test material path (12) extends along a front (11) of the tester (1) and the central ventilation unit (6) is installed in a wall (71) of the turret-like setup which differs from the front (11).

5. A modular tester (1) according to claim 4, wherein the wall (71) in which the central ventilation unit (6) is installed is a rear wall of the turret-like setup which is opposite the front (11).

6. A modular tester (1) according to one of the preceding claims, wherein an air inlet of the at least one central fan (61 to 63) is provided with a grate, screen and/or air filter for preventing a penetration of impurities into the common interior volume (4).

7. A modular tester (1) according to one of the preceding claims, wherein the central ventilation unit (6) comprises several central fans (61 to 63).

8. A modular tester (1) according to one of the preceding claims, wherein
the tester (1) has a basic infrastructure for providing a basic functionality, and
the turret-like setup comprises a base housing (2) for the basic infrastructure and an optional housing (3) which is positioned on the base housing (2) for optional functional modules (43 to 45).

9. A modular tester (1) according claim 8, wherein the basic infrastructure for providing the basic functionality comprises at least one conveying device (16) for the textile test material, a suction opening (17) for the textile test material, and a sensor (52) for testing the uniformity of the textile test material.

10. A modular tester (1) according to claim 2 and claim 8 or 9, wherein all functional modules (41 to 45) of the turret-like setup form a single ventilation group and the central ventilation unit (6) comprises two central fans (61, 62) arranged in a region of the base housing (2) and a third central fan (63) arranged in a region of the optional housing (3).

11. A modular tester (1) according to one of the preceding claims, wherein the turret-like setup comprises air outlet openings leading from the common interior volume (4) to the outside, by means of which the excess air pressure is buildable up by the at least one central fan (61 to 63) in the common interior volume (4) is removable, so that partial air flows directed to the outside are producible at the air outlet openings.

12. A modular tester (1) according to claim 11, wherein at least a part of the air outlet openings lies in the test material path (12) in order to prevent dust deposits in the test material path (12) and on the functional modules (41 to 45) by means of the outwardly directed partial air flows.

## Revendications

1. Appareil de contrôle modulaire (1) pour le contrôle de matière textile allongée à contrôler telle qu'un fil, une mèche ou un ruban de fibres, lequel appareil de contrôle (1) comprend
un trajet de la matière à contrôler (12) et
une superstructure en forme de tour avec plusieurs modules fonctionnels (41-45) disposés les uns à la suite des autres sur le trajet de la matière à contrôler (12) pour mesurer différents paramètres de la matière à contrôler,
**caractérisé en ce que**
plusieurs modules fonctionnels (41-45) sont reliés directement les uns aux autres à l'intérieur de la superstructure en forme de tour de façon à conduire un gaz, de sorte qu'ils forment ensemble un groupe de ventilation à l'intérieur duquel il existe un volume intérieur commun (4), et
le groupe de ventilation présente une unité de ventilation centrale (6) qui est conçue pour ventiler tous les modules fonctionnels (41-45) du groupe de ventilation et qui contient au moins un ventilateur central (61-63) pour former une surpression d'air dans le volume intérieur commun (4).

2. Appareil de contrôle modulaire (1) selon la revendication 1, dans lequel tous les modules fonctionnels (41-45) de la superstructure en forme de tour forment un seul groupe de ventilation.

3. Appareil de contrôle modulaire (1) selon l'une des revendications précédentes, dans lequel l'unité de ventilation centrale (6) se trouve dans le volume intérieur commun (4).

4. Appareil de contrôle modulaire (1) selon l'une des revendications précédentes, dans lequel le trajet de la matière à contrôler (12) passe le long d'une face avant (11) de l'appareil de contrôle (1) et l'unité de ventilation centrale (6) est intégrée dans une paroi (71) de la superstructure en forme de tour différente de la face avant (11).

5. Appareil de contrôle modulaire (1) selon la revendication 4, dans lequel la paroi (71) dans laquelle est intégrée l'unité de ventilation centrale (6) est une paroi arrière de la superstructure en forme de tour faisant face à la face avant (11).

6. Appareil de contrôle modulaire (1) selon l'une des revendications précédentes, dans lequel une entrée d'air de l'au moins un ventilateur central (61-63) est munie d'une grille, d'une crépine et/ou d'un filtre à air pour empêcher la pénétration d'impuretés dans le volume intérieur commun (4).

7. Appareil de contrôle modulaire (1) selon l'une des revendications précédentes, dans lequel l'unité de ventilation centrale (6) présente plusieurs ventilateurs centraux (61-63).

8. Appareil de contrôle modulaire (1) selon l'une des revendications précédentes, dans lequel
l'appareil de contrôle (1) présente une infrastructure de base assurant une fonction de base et
la superstructure en forme de tour comprend un boîtier de base (2) pour l'infrastructure de base et un boîtier d'options (3) posé sur le boîtier de base (2) pour des modules fonctionnels (43-45) en option.

9. Appareil de contrôle modulaire (1) selon la revendication 8, dans lequel l'infrastructure de base comprend, pour réaliser la fonction de base, au moins une installation de transport (16) pour la matière textile à contrôler, une ouverture d'aspiration (17) pour la matière textile à contrôler et un capteur (52) pour le contrôle d'uniformité de la matière textile à contrôler.

10. Appareil de contrôle modulaire (1) selon la revendication 2 et la revendication 8 ou 9, dans lequel les modules fonctionnels (41-45) de la superstructure en forme de tour forment un seul groupe de ventilation et l'unité de ventilation centrale (6) présente deux ventilateurs centraux (61, 62) disposés dans une zone du boîtier de base (2) et un troisième ventilateur central (63) disposé au niveau d'un boîtier d'options (3).

11. Appareil de contrôle modulaire (1) selon l'une des revendications précédentes, dans lequel la superstructure en forme de tour présente des ouvertures de sortie d'air menant vers l'extérieur à partir du volume intérieur commun (4), par lesquelles la surpression d'air créée par l'au moins un ventilateur central (61-63) dans le volume intérieur commun (4) peut être réduite de sorte que des flux d'air partiels dirigés vers l'extérieur peuvent être produits au niveau des ouvertures de sortie d'air.

12. Appareil de contrôle modulaire (1) selon la revendication 11, dans lequel au moins une partie des ouvertures de sortie d'air se trouvent dans le trajet de la matière à contrôler (12) afin d'empêcher, grâce aux flux d'air partiels dirigés vers l'extérieur, le dépôt de poussière dans le trajet de la matière à contrôler (12) et sur les modules fonctionnels (41-45).
